## Europäisches Patentamt

## European Patent Office

## Office européen des brevets

(11) Veröffentlichungsnummer: **0 384 283**
**A2**

(12) **EUROPÄISCHE PATENTANMELDUNG**

(21) Anmeldenummer: 90102872.0

(22) Anmeldetag: **14.02.90**

(51) Int. Cl.⁵: **A61K 47/18, A61K 47/42, A61K 9/02**

(30) Priorität: **22.02.89 DD 325946**

(43) Veröffentlichungstag der Anmeldung:
**29.08.90 Patentblatt 90/35**

(84) Benannte Vertragsstaaten:
**AT BE CH DE FR GB IT LI NL SE**

(71) Anmelder: **VEB Berlin-Chemie**
**Glienicker Weg 125-127**
**DDR-1199 Berlin(DD)**

(72) Erfinder: **Kossowicz, Joachim**
**Kalkbergeweg 79**
**DDR-1165 Berlin(DD)**
Erfinder: **Milde, Karl, Dr.**
**--**
**verstorben(DD)**
Erfinder: **Hacker, Elke, Dr.**
**Ahornstrasse 32**
**DDR-1167 Berlin(DD)**
Erfinder: **Gabel-Tählmann, Vera**
**Eichbuschallee 3**
**DDR-1195 Berlin(DD)**
Erfinder: **Krause, Diana**
**Hönower Strasse 101**
**DDR-1147 Berlin(DD)**
Erfinder: **Strätling, Ernst-Josef, Dr.**
**Lindenstrasse 16**
**DDR-1603 Schulzendorf(DD)**
Erfinder: **Till, Lothar, Dr.**
**Rummelsburger Strasse 47f**
**DDR-1136 Berlin(DD)**

(74) Vertreter: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Arzneimittel zur rektalen Applikation und Verfahren zu ihrer Herstellung.**

(57) Biologisch aktive Substanzen, Resorption, Aminosäure-Fettsäure-Kondensationsprodukt, Suppositorien, Verfahren, Wirkungssteigerung, Arzneimittel, nativ, synthetisch, Hydrolyse, Chloramphenicol

Die Erfindung betrifft wirkungsgesteigerte Arzneimittel zur rektalen Applikation und Verfahren zu ihrer Herstellung. Sie sind dadurch gekennzeichnet, daß als resorptionssteigernde Komponente ein Aminosäure-Fettsäure-Kondensationsprodukt Verwendung findet. Die Erfindung findet besonders Anwendung für den Wirkstoff Chloramphenicol.

## Arzneimittel zur rektalen Applikation und Verfahren zu ihrer Herstellung

Die Erfindung betrifft die Wirkungssteigerung biologisch aktiver Substanzen, die sich durch schlechte oder langsame Resorption bei rektaler Applikation darstellen und keine Peptidstruktur aufweisen.

Es ist bekannt, daß pharmazeutische Wirkstoffe, insbesondere Analgetika, Antiphlogistika, Antirheumatika wie Propiophenazon, Indomethazin, aber auch Chemotherapeutika in Form von Suppositorien zur Anwendung gelangen, weil die orale Applikation zu nachteiligen Nebenwirkungen für den Patienten führt. Nachteil dieser Verfahrensweise für einige der Substanzen ist jedoch ihre unzureichende bzw. verzögerte Resorbierbarkeit aus dem Rektum. Dem wird dadurch begegnet, daß der Wirkstoff im Suppositorium erheblich höher dosiert wird, um im Vergleich zur per-os-Applikation zu annähernd analogen Ergebnissen zu gelangen. Der Einsatz von resorptionsfördernden Stoffen ist bisher bei der rektalen Anwendung von Peptid-Wirkstoffen beschrieben worden, da diese Wirkstoffe meistens im Darm nicht beständig sind und daher sehr schnell in den Blutkreislauf gelangen müssen.

Ziel der Erfindung ist es, durch eine Verbesserung der Resorption biologisch aktiver Substanzen aus dem Rektum den Wirkstoffeinsatz zu senken, die rektale Resorption überhaupt zu ermöglichen, bzw. die Resorption mit dem Ziel des schnelleren Wirkungseintritts zu beschleunigen.

Aufgabe der Erfindung ist das Finden eines Weges, diese Überdosierung des Wirkstoffes herabzusetzen. Als Wirkstoffe werden hier vorzugsweise Chloramphenicol und Propyphenazin genannt. Die Resorptionsverbesserung wird dadurch erzielt, daß als resorptionssteigernde Komponente ein Aminosäure-Fettsäure-Kondensationsprodukt, wobei die Aminosäure auch ein niedermolekulares natives hydrolytisch oder synthetisch gewonnenes Polypeptid sein kann, eingesetzt wird. Die Fettsäurekomponente weist zumeist eine Kettenlänge von $C_{10}$ bis $C_{20}$ auf, wobei dem Bereich $C_{12}$ bis $C_{18}$ der Vorzug gegeben wird. Die resorptionssteigernde Komponente wird in Konzentrationen von 0,1 bis 20 %, auf die Gesamtmasse bezogen, eingesetzt. Als biologisch aktive Substanzen können z.B. Chloramphenicol oder Propyphenazin eingesetzt werden.

Es war überraschend, daß außer einer positiven Beeinflussung der resorbierbaren Menge der aktiven biologischen Substanz auch in Abhängigkeit von der Konzentration der resorptionssteigernden Komponente die Resorptionsgeschwindigkeit beeinflußt werden konnte.

Ausführungsbeispiele

Beispiel 1

Männlichen Kaninchen (Widderbastarde) mit einem durchschnittlichen Gewicht von 3,8 kg, gehalten unter standardisierten, konventionellen Umweltbedingungen, wurde 16 Stunden nach Futterentzug ein Chloramphenicol-Zäpfchen eingeführt und der After für 90 Minuten mit einem Gummiband verschlossen.

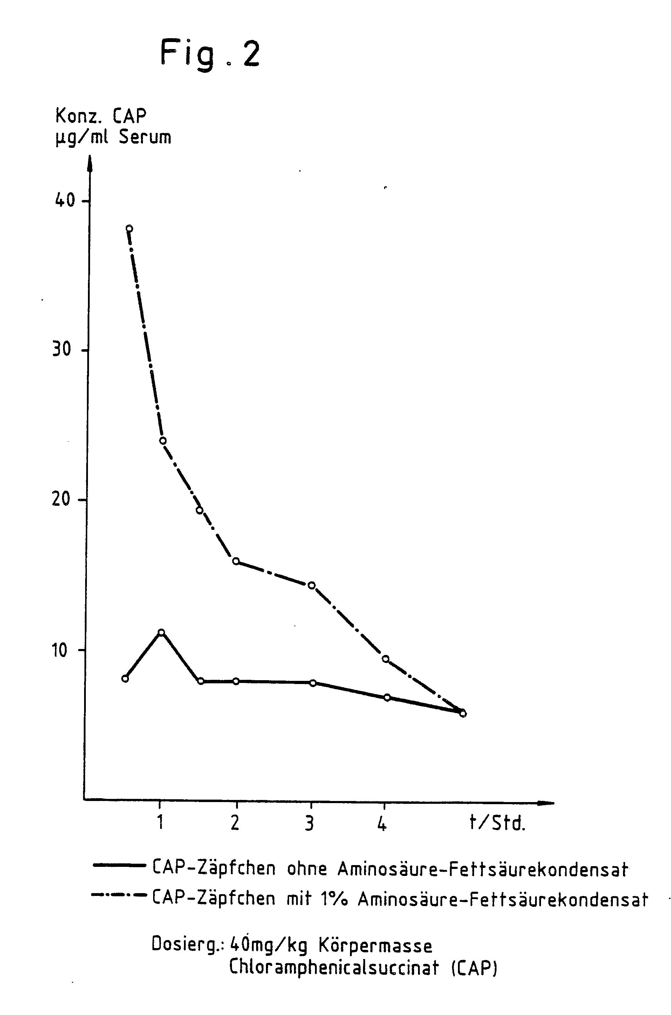

Das Chloramphenicol wurde quantitativ im Blutserum nach der Methode von BESSMANN und STEPHENS (1950) bestimmt. (Lit.: BESSMANN und STEPHENS, S., A colorimetric Method for the Determination of chloromycetin in Serum or Plasma, J. Lab. clin. Med. 35, S. 129, 1950) (Fig. 2).

Beispiel 2

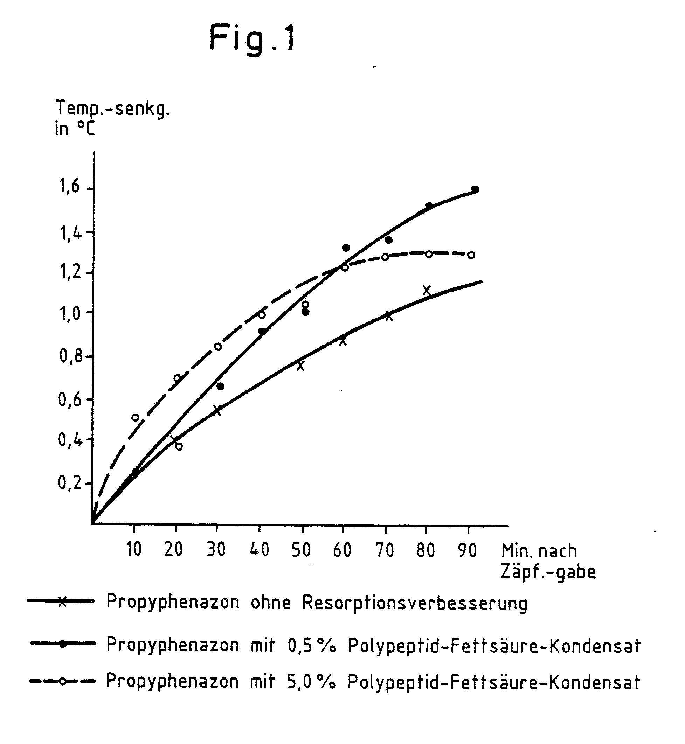

Klinisch gesunden Kaninchen im Gewicht von 2,5 bis 3,5 kg, gehalten unter standardisierten Umweltbedingungen, wurde Pyrecol (gereinigtes Lipopolysaccharid) i.v. injiziert, was zu Fieber führte. Die Temperatursenkung durch Propyphenazinzäpfchen (150 mg) mit und ohne Resorptionsbeeinflussung wurde verglichen (Fig. 1).

## Ansprüche

1. Arzneimittel mit verstärkter Wirkung zur rektalen Applikation, die als biologisch aktive Substanzen keine Peptide enthalten, dadurch gekennzeichnet, daß die Arzneimittel neben der biologisch aktiven Substanz eine resorptionssteigernde Komponente von 0,1 bis 20 % aus Aminosäure-Fettsäure-Kondensationsprodukt aus einfachen oder peptidisch kondensierten Aminosäuren und Fettsäuren der Kettenlänge $C_{10}$ bis $C_{20}$, vorzugsweise $C_{12}$-$C_{18}$, enthalten.

2. Arzneimittel nach Anspruch 1, dadurch gekennzeichnet, daß als biologisch aktive Substanz Chloramphenicol enthalten ist.

3. Verfahren zur Herstellung von Arzneimitteln zur rektalen Applikation mit gesteigerter Wirkung von biologisch aktiven Substanzen, die keine Peptide darstellen, dadurch gekennzeichnet, daß die biologisch aktive Substanz mit einer resorptionssteigernden Komponente von 0,1 bis 20 % aus einem Aminosäure-Fettsäure-Kondensationsprodukt, das aus einfachen oder peptidisch kondensierten Aminosäuren und Fettsäuren der Kettenlän-

ge $C_{10}$ bis $C_{20}$ besteht, eingesetzt wird.

4. Verfahren nach Anspruch 3, dadurch gekennzeichnet, daß das Aminosäure-Fettsäure-Kondensationsprodukt aus einfachen oder peptidisch kondensierten Aminosäuren und Fettsäuren der Kettenlänge $C_{12}$ bis $C_{18}$ besteht.

5. Verfahren nach Anspruch 3 und 4, dadurch gekennzeichnet, daß die peptidisch kondensierten Aminosäure-Ausgangsprodukte sowohl nativen als auch synthetischen Ursprungs sind bzw. durch Hydrolyse aus nativem Material gewonnen werden.

6. Verfahren nach Anspruch 3 bis 5, dadurch gekennzeichnet, daß als biologisch aktive Substanz Chloramphenicol eingesetzt wird.

# Fig.1

Temp.-senkg. in °C

1,6
1,4
1,2
1,0
0,8
0,6
0,4
0,2

10  20  30  40  50  60  70  80  90  Min. nach Zäpf.-gabe

—×— Propyphenazon ohne Resorptionsverbesserung

—●— Propyphenazon mit 0,5 % Polypeptid-Fettsäure-Kondensat

—○—— Propyphenazon mit 5,0 % Polypeptid-Fettsäure-Kondensat

# Fig. 2

Konz. CAP
µg/ml Serum

———— CAP-Zäpfchen ohne Aminosäure-Fettsäurekondensat

—·—·— CAP-Zäpfchen mit 1% Aminosäure-Fettsäurekondensat

Dosierg.: 40mg/kg Körpermasse
Chloramphenicalsuccinat (CAP)